(19) Europäisches Patentamt
European Patent Office
Office européen des brevets

(11) **EP 1 525 006 B1**

(12) **FASCICULE DE BREVET EUROPEEN**

(45) Date de publication et mention
de la délivrance du brevet:
**26.11.2008 Bulletin 2008/48**

(21) Numéro de dépôt: **03756517.3**

(22) Date de dépôt: **30.07.2003**

(51) Int Cl.:
*A61L 12/08* (2006.01)   *C11D 3/00* (2006.01)

(86) Numéro de dépôt international:
**PCT/FR2003/002412**

(87) Numéro de publication internationale:
**WO 2004/014443 (19.02.2004 Gazette 2004/08)**

(54) **SOLUTION DE RINCAGE POUR LENTILLES DE CONTACT**

SPÜLLÖSUNG FÜR KONTAKTLINSEN

RINSING SOLUTION FOR CONTACT LENSES

(84) Etats contractants désignés:
**AT BE BG CH CY CZ DE DK EE ES FI FR GB GR HU IE IT LI LU MC NL PT RO SE SI SK TR**

(30) Priorité: **02.08.2002 FR 0209913**

(43) Date de publication de la demande:
**27.04.2005 Bulletin 2005/17**

(73) Titulaire: **Laboratoire de la Mer**
**35400 Saint-Malo (FR)**

(72) Inventeurs:
• **YVIN, Jean-Claude**
**F-35400 Saint-Malo (FR)**
• **LEROY, Didier**
**F-35730 Pleurtuit (FR)**
• **RAT, Patrice**
**F-75012 Paris (FR)**
• **WARNET, Jean-Michel**
**F-75016 Paris (FR)**

(74) Mandataire: **Koch, Gustave**
**Cabinet Plasseraud**
**52 rue de la Victoire**
**75440 Paris Cedex 09 (FR)**

(56) Documents cités:
**FR-A- 2 803 205      US-A- 5 474 700**

**Description**

L'invention a pour objet une solution de rinçage pour lentilles de contact.

Parmi les lentilles de contact que l'on trouve dans le commerce, les plus utilisées sont celles qui sont établies en des matériaux hydrophiles.

Entre deux utilisations successives, ces lentilles de contact sont rincées à l'aide de solutions notamment aqueuses qui permettent de les désinfecter ; pendant la nuit, ces lentilles sont conservées en milieu humide.

Il existe de nombreuses solutions de rinçage de ce genre.

De par leur caractère hydrophile, ces lentilles absorbent des quantités non négligeables de ces solutions de rinçage, notamment pendant la nuit lorsqu'elle sont conservées immergées dans ces solutions.

Lors de leur mise en place sur l'oeil, elles amènent donc les solutions en question à la surface oculaire, au contact de laquelle elles peuvent être relarguées. Et il est évident que lorsqu'elles comportent des constituants irritants pour l'oeil, le patient porteur des lentilles peut développer des intolérances telles qu'il doive abandonner l'usage des lentilles de contact.

Or, c'est précisément ce que l'on observe dans la pratique ; les intolérances aux lentilles de contact sont de plus en plus nombreuses et un certain pourcentage de ces intolérances est très probablement dû à la présence des constituants irritants dont il vient d'être question.

Le but de l'invention est donc surtout de remédier aux inconvénients de l'art antérieur et de mettre à la disposition de l'utilisateur une solution de rinçage pour lentilles de contact ne comportant pas de constituants irritants pour l'oeil.

Et la Société Demanderesse a le mérite d'avoir trouvé qu'une solution répondant aux desiderata exposés ci-dessus est une solution ionique aqueuse obtenue à partir de l'eau de mer dont la composition ionique est du point de vue qualitatif celle de l'eau de mer et du point de vue quantitatif telle que d'une part son pH soit de 4 à 9, de préférence de 7 à 8 et que d'autre part son osmolalité soit de 150 à 700, de préférence de 250 à 350 mOsm/kg.

Plus particulièrement, il s'agit d'une solution ionique aqueuse obtenue à partir de d'eau de mer caractérisée par :

- une valeur de pH de préférence inférieure ou au plus égale aux valeurs les plus basses du pH de l'eau de mer,
- une osmolalité inférieure à celle de l'eau de mer et
- une composition du point de vue ionique qui est qualitativement et quantitativement celle de l'eau de mer, à l'exception du point de vue quantitatif, d'une part, de la concentration en potassium qui est supérieure à celle de l'eau de mer et, d'autre part, des concentrations en Na, Mg, Ca et Cl qui sont inférieures à celles de l'eau de mer, lesdites concentrations étant

    o. pour Na*, de 1300 à 1500, de préférence de 500 à 1000 mg/l,
    o pour K*, de 4500 à 6500, de préférence de 5000 à 6000 mg/l,
    o pour Mg**, de 50 à 1300, de préférence de 100 à 500 mg/l,
    o pour Ca**, de 20 à 350, de préférence de 40 à 200 mg/l,
    pour Cl*, de 4000 à 6000, de préférence de 4500 à 5000mg/l.

La solution ionique aqueuse en question, fait l'objet de la demande de brevet français n° 99 16814 déposée le 31 décembre 1999.

L'invention a donc pour objet l'utilisation, en tant que solution de rinçage pour lentilles de contact, de la susdite solution ionique aqueuse.

La supériorité de la solution utilisée conformément à l'invention par rapport aux solutions de rinçage pour lentilles de contact qui existent déjà a été démontré par les essais "in vitro" décrits ci-après.

Ces essais ont été effectués en utilisant des cellules issues d'une lignée de cellules conjonctivales humaines dite Wong Kilbourn Derwated Conjonctival Cell (WKD, ATTCC 20.2).

Ces cellules ont été mises au contact à 100%, c'est à dire immergées, pendant au moins 15 minutes d'une part dans la solution utilisée conformément à l'invention et d'autre part dans quatre solutions de rinçage pour lentilles de contact du commerce commercialisées sous les marques de fabrique "Complete", "SoloCare", Opti Free" et "Renu" respectivement par les laboratoires Allergan, Ciba Vision, Alcon et Bausch et Lomb.

Les effets produits sur ces cellules conjonctivales ont été évalués par différents tests désignés par "MIFALC" (Microtitration Fluorimetric Assays on live Cells) dans lesquels on utilise ce qu'on appelle des "sondes" fluorescentes appliquées directement sur des cellules vivantes adhérentes, à savoir

- le test au rouge neutre
- le test au Hoechst 33342 ou HO/Iodure de propidium ou IP
- le test à l'H2DCF-DA
- le test à l'hydroethydine

- le test à la Rhodamine 123
- le test au Nonyl Acridine Orange (NAO)

qui, respectivement permettent de connaître l'effet des solutions testées sur les marqueurs cellulaires suivants :

- la viabilité cellulaire,
- la condensation de la chromatine(apoptose),
- les formes réactives de l'oxygène,
- l'anion superoxyde,
- le potentiel transmembranaire mitochondrial,
- la masse mitochondriale

Les cellules Wong Kilbourn Derivated Conjonctival Cell (WKD, ATCC 20.2) sont cultivées en flasques de 75 $cm^2$ dans un incubateur thermostaté à 37°C sous atmosphère humide contenant 5% de $CO_2$.

Le milieu de culture utilisé est du DMEM enrichi à 10% en SVK, contenant 1% de glutamine et des antibiotiques, notamment de l'ampicilline/Kanamycine.

Le milieu de culture est renouvelé tous les 2-3 jours, les cellules sont trypsinées à 80% de confluence soit 1 fois par semaine en moyenne.

Les tests sont effectués dans des microplaques à 96 puits.

24 heures avant le traitement des cellules testées à l'aide des différentes solutions de rinçage, les microplaques 96 puits sont ensemencées avec les cellules WKD à raison de 5 000 cellules par puits.

On utilise 1 plaque par test, soit 8 plaques au total.

Etant donné que les sondes fluorescentes émettent dans l'UV, il est nécessaire d'utiliser des microplaques spéciales à bords noirs (notamment dans le test au Hoechst 33342- Ho/IP).

24 heures après l'ensemencement, les microplaques sont traitées avec les solutions à tester.

L'incubation est de 15 minutes et s'effectue selon le schéma de plaque suivant comportant les colonnes 1, 2, 3, 4, 5, 6, 7, 8, 9, 10, 11 et 12 et les lignes A, B, C, D, E, F, G, et H.

|   | 1 | 2 | 3 | 4 | 5 | 6 | 7 | 8 | 9 | 10 | 11 | 12 |
|---|---|---|---|---|---|---|---|---|---|----|----|----|
| A |   |      |      |      |      |      |      |      |      |      |      |   |
| B |   | Tem  | Tem  | Tem  | Tem  | Tem  | Tem  | Tem  | Tem  | Tem  | Tem  |   |
| C |   | Sero | Sero | Sero | Sero | Sero | Sero | Sero | Sero | Sero | Sero |   |
| D |   | Comp | Comp | Comp | Comp | Comp | Comp | Comp | Comp | Comp | Comp |   |
| E |   | Opti | Opti | Opti | Opti | Opti | Opti | Opti | Opti | Opti | Opti |   |
| F |   | Renu | Renu | Renu | Renu | Renu | Renu | Renu | Renu | Renu | Renu |   |
| G |   | Solo | Solo | Solo | Solo | Solo | Solo | Solo | Solo | Solo | Solo |   |
| H |   |      |      |      |      |      |      |      |      |      |      |   |

dans lequel les différentes abréviations signifient :

Tem: temoin = BSS
Comp: Complete
Renu : Renu
Sero: Solution utilisée conformément à l'invention
Opti : Optifree
Solo: Solo care

Les solutions sont utilisées pures et sous conditions stériles.

L'expérimentation est renouvelée trois fois de suite avec ce même protocole.

Dans les différents tests, les effets obtenus se traduisent par une fluorescence plus ou moins intense.

On décrit ci-après et dans un premier temps la façon dont sont mis en oeuvre les différents tests MIFALC dont il a été question plus haut.

### 1. Test au Rouge Neutre

Dans ce test, on évalue l'effet des solutions testées sur la viabilité cellulaire et l'intégrité membranaire, les résultats étant exprimés en % de fluorescence par rapport au témoin.

Ces résultats peuvent être analysés à l'aide du logiciel statistique "sigmastat32" version 2.0 (Analyse de variance et post hoc test, Test DUNNET)

Le test au Rouge Neutre est réalisé selon le protocole de Borenfreund (qui prévoit 50 $\mu$ml/3h) ; ce protocole a été utilisé et validé dans différentes études multicentriques européennes; il est considéré comme l'un des plus sensibles pour l'évaluation de la cytotoxicité d'une substance.

Le Rouge Neutre est un colorant vital d'inclusion dont la fixation à l'intérieur des cellules est fonction de la viabilité cellulaire et de l'intégrité membranaire.

Il permet ainsi d'évaluer l'effet cytotoxique d'un agent chimique ou physique après 24 à 72 h, en moyenne, de contact.

L'expression du résultat se fait classiquement en pourcentage de viabilité par rapport au témoin non traité.

L'évaluation du résultat du test au Rouge Neutre est faite par détection fluorimétrique pour augmenter la spécificité de la réaction selon une procédure MiFALC (Microtitration Fluorimetnc Assays on Living Cells).

La sensibilité de détection du Rouge Neutre dans ces conditions est de 50pg/ml. (Rat P et aL, Cell Biol. Toxicol., 1994, 10, 329-337; Rat P. et ai., Methods in Enzymology, 1995, 252, 331-340.).

### 2. Test au Hoechst 33342/iodure de propidium

Par ce test, on évalue la condensation de la chromatine (Apoptose), autrement dit le mécanisme de mort cellulaire

(Apoptose/Nécrose->rapport Hoechst/ Rouge Neutre)

Le "Hoechst 33342" utilisé, est une sonde spécifique de l'adénine et de la thymine, qui sont des constituants de l'ADN des cellules.

Un contre-marquage avec du iodure de propidium garantit une fixation du "Hoechst" sur des cellules normales ou seulement en apoptose, car les noyaux ou les débris d'ADN des cellules en nécrose sont colorés très rapidement par l'iodure de propidium intercalant qui empêche alors la fixation ultérieure du "Hoechst".

Une fluorescence bleue foncée est observée après fixation du "Hoechst" sur l' ADN des cellules normales.

Si le nombre de cellules normales diminue, la fluorescence du "Hoechst 33342" décroît.

Mais une condensation de la chromatine associée à un phénomène d'apoptose induit une surexpression de la fluorescence du "Hoechst" et donc une augmentation de fluorescence du signal *I* témoin.

On peut ainsi observer, après traitement de seulement 15 minutes par la solution l'OptiFree une baisse régulière du signal.

Par contre, le signal de fluorescence reste élevé et même augmente par rapport au témoin pour les solutions SOLOCARE ET RENU.

On pourrait en déduire l'hypothèse d'un mécanisme d'apoptose dans la cytotoxicité de ces produits sur notre modèle cellulaire retenu.

### 3. Test à la DCFH-DA (ou H$_2$DCF-DA) et à l'Hydroéthidine

Ces tests permettent d'évaluer une éventuelle production radicalaire ou plus précisément respectivement la synthèse de peroxyde d'hydrogène ou celle d'anions superoxyde.

Les sondes fluorogènes DCFH-DA ou hydroéthydine émettent au contact de radicaux libres (ROS, H$_2$O$_2$, anion superoxyde) une fluorescence verte.

Cette production radicalaire très labile est très sensible aux artefacts des protocoles standards d'évaluation (trypsination, monodispersion des cellules..), d'où l'importance d'utiliser la technologie cytofluorimétrique en lumière froide (MCCM), qui permet d'utiliser ces sondes directement sur des cellules vivantes adhérentes sans trypsination et sans monodispersion des cellules.

La limitation de ces artéfacts permet d'améliorer considérablement la qualité du signal et de pouvoir travailler, avec une grande sensibilité (pg-fg/ml), sur des cellules classiques (fibroblastes, ténocytes, cellules conjonctivales) qui ont un niveau radicalaire base plus faible que des cellules spécialisées (macrophages ou polynucléaires en suspension) utilisées en cytométrie de flux.

Plus particulièrement, le test à la DCFH-DA permet l'évaluation des formes réactives de l'oxygène, principalement le peroxyde d'hydrogène.

La sonde H2 DCF-DA est clivée à l'intérieur des cellules par des estérases cellulaires.

Le dérivé fluorescéine formé H2DCF n'est pas fluorescent, mais peut le devenir au contact de formes réactives de l'oxygène (par exemple du peroxyde d'hydrogène) pour former de la dichlorofluorescéine qui émet une fluorescence verte.

On peut ainsi observer une surproduction de radicaux libres en fonction du temps d'incubation (15, 30, 45,60 minutes) ou en fonction des différents produits testés.

Concernant le test à la l'HydroEthidine on notera que cette dernière, qui n'est pas fluorescente, se transforme en éthidine *(bromure d'éthidium)* fluorescente sous l'effet d'anions superoxyde.

Cette réaction spécifique de l'anion superoxyde permet de mettre en évidence le rôle de ce radical dans certains mécanismes de cytotoxicité.

4. <u>Les tests à la Rhodamine 123 et au NAO</u>

Ces tests permettent d'évaluer l'activité et de la masse mitochondriale globale.

La mitochondrie semble être une cible précoce et privilégiée dans le mécanisme de cytotoxicité de nombreuses molécules.

Jusqu'à présent, l'évaluation de l'activité mitochondriale était délicate et nécessitait un matériel très lourd (Laser en CMF ou microélectrodes).

Les nouvelles méthodes de Microtitration Cytofluorimétrique (MCM), qui incorporent la technologie de fluorimétrie en lumière froide (MCCM), permettent l'utilisation de sondes spécifiques directement sur des cellules vivantes.

Plus particulièrement, le test à la Rhodamine 123 (Rh123) est une sonde bien connue en cytométrie en flux *(CMIF)* pour sa fixation spécifique sur la mitochondrie.

La sonde fluorescente Rhodamine 123 (Rh 123) est donc appliquée à des cellules vivantes pour évaluer les variations de l'activité mitochondriale globale suite à l'action des solutions testées.

La fluorescence de la Rhodamine 123 est proportionnelle au potentiel transmembranaire mitochondrial.

L'évaluation de la Rhodamine 123 est faite avec une détection fluorimétrique (Exc 490 nn./Em *535* nm) selon une procédure MiFALC (Microtitration Fluonmetric Assays on Living Cells).

La sensibilité de détection de la Rhodamine 123 dans ces conditions est de 20 pg/ml (Rat P et ai, Cell Biol. Toxicol.,1994. 10,329-3). L'interprétation des résultats nécessite l'analyse d'un test à la NAO (Nonyl Acridine Orange) associé.

5. <u>Test au Nonyl Acridine Orange</u>

La Nonyl Acridine Orange est une sonde fluorescente dans le rouge et dont la fluorescence est indépendante du potentiel transmembranaire.

En effet, la NAO se fixe sur le cardiolipide de la membrane mitochondriale. Plus la masse et la surface mitochondriale sont importantes plus la fixation du NAO sera importante et sa fluorescence élevée.

Lors d'un stress cellulaire, les mitochondries peuvent grossir ou diminuer en taille induisant indirectement une modification du potentiel transmembranaire mitochondrial.

Donc, l'analyse des tests Rhodamine 123 et NAO permet une évaluation fine de l'activité mitochondriale (test à la Rhodamine 123) qui peut être pondérée par les modifications de la masse mitochondiale (test NAO) suite à un stress cellulaire.

Autrement dit, elle permet d'avoir une idée précise sur l'effet produit par les solutions testées.

Ainsi que l'on vient de le voir, les tests retenues donnent lieu à des signaux de fluorescence.

Il s'agit donc d'évaluer ces signaux de fluorescence.

Pour ce faire, on a recours à une technique de Microtitration Cytofluorimétrique (MCM) incorporant la technologie de fluorimétrie en lumière froide (MCCM: Microplate Cold light Cytometry) décrite par Rat P. et al., dans Methods in Enzymology,1995. 252,331-340.

La limite de détection de cette technique est : pico-femtogramme de sonde/ml

Le spectre de détection est : 280 à 870 mn

Il est ainsi possible d'effectuer des mesures de sondes fluorescentes intracellulaires directement en microplaques sur des cellules vivantes (MIFALC tests : Microtitration Fluorimetric Assay on Living Cells)

Grâce à cette technique, il devient possible d'étudier la cytotoxicité sur des temps courts et longs ainsi que des cinétiques en continu, car la lignée cellulaire utilisée a un équipement enzymatique stable dans le temps à la différence de la primoculture ; de plus la technologie utilisée permet l'évaluation de la cinétique de fluorescence sur des cellules adhérentes.

Elle permet également d'évaluer la cytotoxicité directement sur des cellules vivantes adhérentes (tests M1FALC -Microtitration Fluorimetric Assays on Live Cells), car le fait de trypsiner ou de monodisperser les cellules pour permettre leur utilisation en immunochimie ou en cytométrie de flux induit différents artéfacts qui peuvent altérer l'observation et l'interprétation correcte de certains marqueurs labiles (par exemple des radicaux libres...).

Les cellules de l'oeil sont des cellules adhérentes qui perdent certaines fonctions intracellulaires si on les sépare de leur support ou si on les dissocie l'une de l'autre.

Il est donc préférable de travailler sur des cellules vivantes adhérentes en utilisant les procédures MiFALC.

Enfin, l'utilisation d'une technologie cytofluorimétrique sur microplaques présente l'avantage d'associer la spécificité de détection des méthodes fluorimétriques, la sensibilité d'une technologie en lumière froide (pico-femtog/ml de sonde détectée) et la reproductibilité de méthodes sur microplaques.

L'interprétation des résultats des tests venant d'être décrits, on peut

- d'évaluer le type de mort cellulaire induit (nécrose ou apoptose),
- d'évaluer les formes radicalaires de l'oxygène ($H_2O_2$) induits,
- d'évaluer une éventuelle surproduction d'anion superoxyde et
- d'évaluer l'intensité de l'activité mitochondriale.

a) Evaluation du type de mort cellulaire (nécrose-apoptose)

L'association de plusieurs marqueurs fluorescents permet la mesure simultanée des mécanismes de mort cellulaire de type nécrose et apoptose.

Pour ce faire, on utilise l'association d'une sonde telle que le Rouge Neutre pour mesurer l'intégrité membranaire avec deux sondes ADN, Hoechst 33342 et Iodure de propidium, pour la mesure des mécanismes d'apoptose et de condensation de la chromatine.

Une augmentation significative du "Hoechst" par rapport au témoin est généralement le signe d'une condensation de la chromatine lors des mécanismes d'apoptose qui est d'ailleurs confirmée par les observations au microscope en fluorescence.

On peut ainsi observer que la solution utilisée conformément à l'invention est globalement inerte et ne donne pas de cytotoxicité particulière que ce soit pour la nécrose ou l'apoptose.

Par contre, les solutions du commerces telles que le Rénu® ou le Solo care® induisent une augmentation significative de la fluorescence du "Hoechst", signe d'un stress cellulaire de type apoptose.

Pour l'Optifree®, en moins de 15 minutes, on observe une mort cellulaire de type nécrose; cette nécrose peut en fait être un mécanisme d'apopto-nécrose qui pourrait être précisé avec les tests complémentaires.

Dans le cas du produit Complete®, on observe un stress cellulaire avec augmentation des deux marqueurs dont le mécanisme pourra être précisé par les autres tests.

De ce fait, dès cette première série de tests, on observe deux groupes, d'une part la solution utilisée selon l'invention qui est globalement inerte sur les cellules conjonctivales de l'oeil humain, et, d'autre part, les différents produits multifonctions du commerce pour le rinçage des lentilles de contact qui induisent toutes un stress cellulaire de type nécrose, apopto-nécrose ou apoptose.

b) Evaluation des formes radicaiaires de l'oxygène (1120)

On observe une cinétique d'apparition des formes radicalaires de l'oxygène.

Cette cinétique correspond aux tests effectués avec la sonde H2DCF-DA, qui réagit avec les différentes formes radicalaires de l'oxygène, et particulièrement avec le peroxyde d'hydrogène ($H_2O$).

On peut ainsi observer un comportement extrêmement différent entre la solution conformément à l'invention et les produits du commerce.

Après 15 minutes de contact, il n'y a pas de différence significative entre les différentes solution connues du commerce.

Il ne semble donc pas y avoir de rôle spécifique des formes radicalaires de l'oxygène dans l'induction des stress cellulaires observés précédemment sur des temps courts de 15 minutes.

Par contre, le déclenchement de ces mécanismes d'apoptose et de nécrose peut induire des modifications de la production de formes radicalaires.

Ainsi, après 60 minutes de contact, toutes les solution du commerce induisent une diminution des formes radicalaires de l'oxygène probablement liée à un effet de stress cellulaire et même à une diminution du nombre des cellules, due aux morts cellulaires observées dès les 15 premières minutes (nécrose et apoptose) vues précédemment avec les tests Rouge Neutre et "Hoechst".

Dans le cas de la solution utilisée conformément à l'invention, on observe que les cellules sont fonctionnelles et réagissent à cet agent exogène par une surproduction radicalaire sans induire d'altération significative de la cellule car les tests de cytotoxicité globale (tests au Rouge Neutre ne sont pas perturbés.

### c) Evaluation d'une surproduction d'anion superoxyde

On observe une surproduction d'anion superoxyde suite au traitement par les différentes solutions testées.

On observe, en fait, qu'il n'y a pas de différence significative par rapport au témoin et l'on n'observe pas de surproduction d'anion superoxyde quels que soient les produits. A noter que la production d'anion superoxyde dans les cellules incubées avec la solution utilisée conformément à l'invention a tendance à être la plus faible des produits testés sans observer de différence significative au vu des écarts-types importants.

On observe donc globalement un stress cellulaire important s'orientant vers la mort cellulaire par apoptose mais sans être lié à un stress radicalaire significatif.

Des tests complémentaires sur la mitochondrie permettront d'explorer les autres voies d'induction de l'apoptose.

### d) Evaluation de l'activité mitochondriale

Pour cette évaluation, on associe simultanément deux sondes fluorescentes.

La première de ces sondes à savoir la sonde Rhodamine 123, permet d'évaluer le potentiel transmembranaire mitochondrial, alors que la deuxième, NAO (nonyl acridine orange), permet de mesurer la masse mitochondriale.

On observe ainsi, après seulement 15 minutes de contact, une diminution significative du potentiel transmembranaire mitochondrial pour les solutions du commerce de type Rénu® ou Solocare®.

On sait qu'une telle diminution du potentiel transmembranaire mitochondrial apparaît dans des phases précoces d'apoptose qui ont été observées dès 15 minutes et est confirmée par l'apparition de la condensation de la chromatine avec les sondes telles que le "Hoechst".

En outre, on observe une dissociation de la masse mitochondriale et de l'activité mitochondriale pour les solutions du commerce telles que le Complete® et l'Optifree® ce qui confirme le stress cellulaire observé auparavant.

La solution utilisée conformément à l'invention, quant à elle, apparaît globalement inerte comparée aux autres solutions du commerce utilisées pour le rinçage des lentilles de contact.

Globalement, il apparaît donc que tous les produits du commerce induisent un stress cellulaire significatif.

Contrairement à la solution utilisée conformément à l'invention, le Solocare® et le Rénu~ induisent dès 15 minutes d'incubation des mécanismes d'apoptose très nets avec condensation de la chromatine et altération des fonctions mitochondriales.

Les solutions Optifree@ et Complete induisent un stress cellulaire caractérisé par le déclenchement du système de compensation mitochondriale, diminution de la masse, augmentation du potentiel transmembranaire mitochondrial, qui, si le stress perdure, peuvent aboutir à des mécanismes d'apoptose-nécrose.

L'ensemble des constatations et conclusions qui précèdent montrent très nettement la supériorité, de par son innocuité, de la solution utilisée conformément à l'invention par rapport aux solutions déjà existantes pour le rinçage de lentilles de contact.

Et cela d'autant plus que les stress observés et décrits plus haut et qui permettent d'aboutir à ces conclusions ont été observés après seulement 15 minutes de contact avec les solutions en question. Or, en pratique, ces solutions peuvent s'adsorber sur la lentille lors du rinçage et être relargués pendant plusieurs heures après mise en contact avec le globe oculaire du patient.

Du point de vue pratique, les solutions ioniques aqueuses, pour autant qu'elles sont destinées à l'utilisation pour le rinçage des lentilles de contact peuvent être préparées comme indiqué dans la demande de brevet identifiée plus haut dans laquelle on trouvera également d'utiles précisions quant à leur propriétés.

### Revendications

1. Utilisation pour le rinçage des lentilles de contact, notamment celles établies en matériaux hydrophile, d'une solution ionique aqueuse obtenue à partir de l'eau de mer dont la composition ionique est du point de vue qualitatif celle de l'eau de mer et du point de vue quantitatif telle que d'une part son pH soit de 4 à 9, de préférence de 7 à 8 et que d'autre part son osmolalité soit de 150 à 700, de préférence de 250 à 350 mOsm/kg.

2. Utilisation pour le rinçage des lentilles de contact, notamment celles établies en matériaux hydrophile, d'une solution ionique aqueuse selon la revendication 1, **caractérisée par**

   - une valeur de pH de préférence inférieure ou au plus égale aux valeurs les plus basses du pH de l'eau de mer,

- une osmolalité inférieure à celle de l'eau de mer et
- une composition du point de vue ionique qui est qualitativement et quantitativement celle de l'eau de mer, à l'exception du point de vue quantitatif, d'une part, de la concentration en potassium qui est supérieure à celle de l'eau de mer et, d'autre part, des concentrations en Na, Mg, Ca et Cl qui sont inférieures à celles de l'eau de mer, lesdites concentrations étant

  o pour Na*, de 1300 à 1500, de préférence de 500 à 1000 mg/l,
  o pour K*, de 4500 à 6500, de préférence de 5000 à 6000 mg/l,
  o pour Mg**, de 50 à 1300, de préférence de 100 à 500 mg/l,
  o pour Ca**, de 20 à 350, de préférence de 40 à 200 mg/l,
  o pour Cl*, de 4000 à 6000, de préférence de 4500 à 5000mg/l.

**Claims**

1. Use for rinsing contact lenses, in particular those made of hydrophilic materials, of an aqueous ionic solution obtained from sea water the ionic composition of which from the qualitative point of view is that of sea water and from the quantitative point of view is such that on the one hand its pH is from 4 to 9, preferably from 7 to 8 and that on the other hand its osmolality is from 150 to 700, preferably from 250 to 350 mOsm/kg.

2. Use for rinsing contact lenses, in particular those made of hydrophilic materials, of an aqueous ionic solution according to claim 1, **characterized by**:

   - a pH value preferably lower than or at most equal to the lowest pH values of sea water,
   - an osmolality lower than that of sea water and
   - a composition from the ionic point of view which is qualitatively and quantitatively that of sea water, with the exception that from the quantitative point of view, on the one hand, the potassium concentration is higher than that of sea water and, on the other hand, the Na, Mg, Ca and Cl concentrations are lower than those of sea water, said concentrations being

     • for Na*, from 1300 to 1500, preferably from 500 to 1000 mg/l,
     • for K*, from 4500 to 6500, preferably from 5000 to 6000 mg/l,
     • for Mg**, from 50 to 1300, preferably from 100 to 500 mg/l,
     • for Ca**, from 20 to 350, preferably from 40 to 200 mg/l,
     • for Cl*, from 4000 to 6000, preferably from 4500 to 5000 mg/l.

**Patentansprüche**

1. Verwendung einer wässrigen ionischen Lösung, die auf der Basis von Seewasser hergestellt wurde, deren ionische Zusammensetzung aus qualitativer Sicht eine solche von Meerwasser ist und aus quantitativer Sicht eine solche, die einerseits einen pH von 4 bis 9, vorzugsweise von 7 bis 8 aufweist und andererseits eine Osmolalität von 150 bis 700, vorzugsweise von 250 bis 350 mOsm/kg aufweist, zum Ausspülen von Kontaktlinsen, insbesondere solcher, die aus hydrophilen Materialien hergestellt werden.

2. Verwendung einer ionischen wässrigen Lösung nach Anspruch 1 zum Ausspülen von Kontaktlinsen, insbesondere solcher, die aus hydrophilen Materialien hergestellt werden, **dadurch gekennzeichnet, dass**

   - ein pH-Wert von vorzugsweise unterhalb oder auch gleich den Werten des pH von Meerwasser ist,
   - eine Osmolalität unterhalb jener von Meerwasser aufweist und
   - eine Zusammensetzung, welche aus ionischer Sicht qualitativ und quantitativ eine solche von Meerwasser ist, mit der Ausnahme dass in quantitativer Hinsicht einerseits die Konzentration an Kalium oberhalb jener von Meerwasser liegt und andererseits die Konzentrationen an Na, Mg, Ca und Cl unterhalb solcher von Meerwasser liegen, wobei die Konzentrationen sind

     • für Na$^+$ von 1300 bis 1500, vorzugsweise von 500 bis 1000 mg/l,
     • für K$^+$ von 4500 bis 6500, vorzugsweise von 5000 bis 6000 mg/l;
     • für Mg$^{++}$ von 50 bis 1300, vorzugsweise von 100 bis 500 mg/l,

- für Ca$^{++}$ von 20 bis 350, vorzugsweise von 40 bis 200 mg/l, und
- für Cl$^{+}$ von 4000 bis 6000, vorzugsweise von 4500 bis 5000 mg/l.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 9916814

**Littérature non-brevet citée dans la description**

- **RAT P et al.** *Cell Biol. Toxicol.,* 1994, vol. 10, 329-337
- **RAT P.** *Methods in Enzymology,* 1995, vol. 252, 331-340
- **RAT P.** *Cell Biol. Toxicol.,* 1994, vol. 10, 329-3
- **RAT P. et al.** *Methods in Enzymology,* 1995, vol. 252, 331-340